# EUROPEAN PATENT APPLICATION

(11) **EP 4 260 686 A1**
(43) Date of publication of application: **18.10.2023**
(21) Application number: 21915676.7
(22) Date of filing: 23.12.2021
(51) Int. Cl.: A01H 6/82, A01H 1/06, A01H 1/02, C12N 9/02, C12N 15/113, C12N 15/82

(54) **METHOD FOR PROMOTING BETA-CAROTENE OR LUTEIN BY USING TOMATO LYCOPENE EPOXIDASE**

(30) Priority: 28.12.2020 KR 20200184545; 28.12.2020 KR 20200184546
(71) Applicant: Kyungpook National University Industry-Academic Cooperation Foundation, Daegu 41566 (KR)
(72) Inventor: LEE, Je Min, Daegu 42108 (KR)
(74) Representative: BCKIP Part mbB
(86) International application number: PCT/KR2021/019727
(87) International publication number: WO 2022/145878

(57) **Abstract**

The present invention relates to a wild-type-derived introgression line of a tomato in which the expression of BC2.1 is inhibited and the amount of beta-carotene or lutein is increased; and a transgenic tomato.

## Description

### [Technical Field]

The present invention relates to: a wild-type-derived introgression line of a tomato in which the expression of tomato lycopene epoxidase BC2.1 is inhibited and the amount of beta-carotene or lutein is increased; and a transgenic tomato.

### [Background Art]

Carotenoids are organic pigments synthesized from fat and basic organic metabolites by plants, algae, bacteria and fungi, and are also called tetraterpenoids. Carotenoids are also essential and useful nutrients to animals as metabolite precursors (e.g., vitamin A and antioxidants, respectively), and have specific health benefits such as prevention of blindness and maintenance of an immune system. Animals lack the *de novo* synthesis ability of carotenoids and need to consume carotenoids through fruits and vegetables. For this reason, an interest on improved crops with increased carotenoid content and genetic control of carotenoid biosynthesis in plants has been increased. A provitamin A content may be greatly increased by increasing both breeding and sequestration increases through genetic engineering using genetic resources and gene silencing of heterologous or endogenous genes. Studies on beta-carotene and carotenoid biosynthesis leading to its regulation have been actively conducted. A carotenoid biosynthetic pathway from phytoene to lycopene and beta-carotene, and following desaturation, isomerization, and additional cyclization are key to carotenoid accumulation during tomato ripening. Chromoplast specific lycopene β-cyclase (*CYC-B,* Chromoplast specific β-cyclase) is a genetic determinant of beta-carotene accumulation during tomato ripening, and beta-carotene is not completely converted to lycopene by endogenous *CYC-B* overexpression.

Lutein is one of 600 naturally occurring carotenoids known to date. The lutein is synthesized only in plants and other xanthophylls, and is found in a large amount in leafy vegetables such as spinach, kale, and yellow carrots, but general lutein products contain 99% or 99% of higher of flower extracts of marigold. The lutein has been recently judged to be helpful for eye health and approved as an individual authorized ingredient, but the lutein is known to help eye health by maintaining macular pigment density, which may be reduced due to aging. However, since the lutein is not naturally produced in the body, there is a limitation that the lutein needs to be taken from the outside through foods, supplements, or the like.

### [Disclosure]

### [Technical Problem]

The present inventors found the increased content of beta-carotene or lutein in a wild-type-derived introgression line of a tomato in which expression of BC2.1 was inhibited and a transgenic tomato and completed the present invention.

Therefore, an object of the present invention is to provide an introgression line of a tomato in which the expression of BC2.1 is inhibited and a transgenic organism.

### [Technical Solution]

One aspect of the present invention provides a tomato in which the expression of BC2.1 is inhibited and the amount of beta-carotene or lutein is increased.

In an exemplary embodiment of the present invention, the expression of ORF2 of the BC2.1 may be inhibited.

In an exemplary embodiment of the present invention, the expression of the BC2.1 may be inhibited to express *LCY-B,* thereby increasing the content of lutein.

In an exemplary embodiment of the present invention, the expression of the BC2.1 may be inhibited by administering an RNAi construct. The RNAi construct may be prepared using RNAi-F having a sequence represented by SEQ ID NO: 59 and RNAi-R having a sequence represented by SEQ ID NO: 60.

In an exemplary embodiment of the present invention, the expression of the BC2.1 may be inhibited by administering a sequence encoding BC2.1 containing a mutation to the tomato. The sequence encoding BC2.1 containing the mutation may be selected from nucleic acid sequences represented by SEQ ID NOs: 2 to 4.

In an exemplary embodiment of the present invention, the tomato may be obtained by crossing subII,2-2-1, a genetic introgression line of Solanum. pennellii LA716, with IL6-3, or produced by crossing subIL2-2-1 with IL12-2.

### [Advantageous Effects]

According to the present invention, it is possible to increase the content of beta-carotene or lutein by inhibiting the expression of BC2.1, and to genetically improve the nutrients of many crops. In addition, it is possible to understand the genetic and biochemical basis of accumulation of plant carotenoids by identifying lycopene epoxidase BC2.1 and to provide a molecular tool for screening for allelic variants or genetic variants for increased provitamin A and lutein.

### [Description of Drawings]

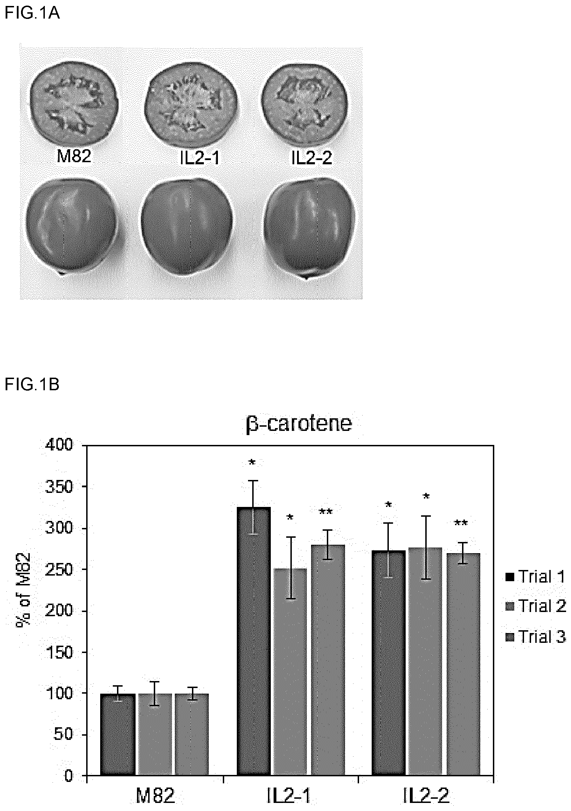

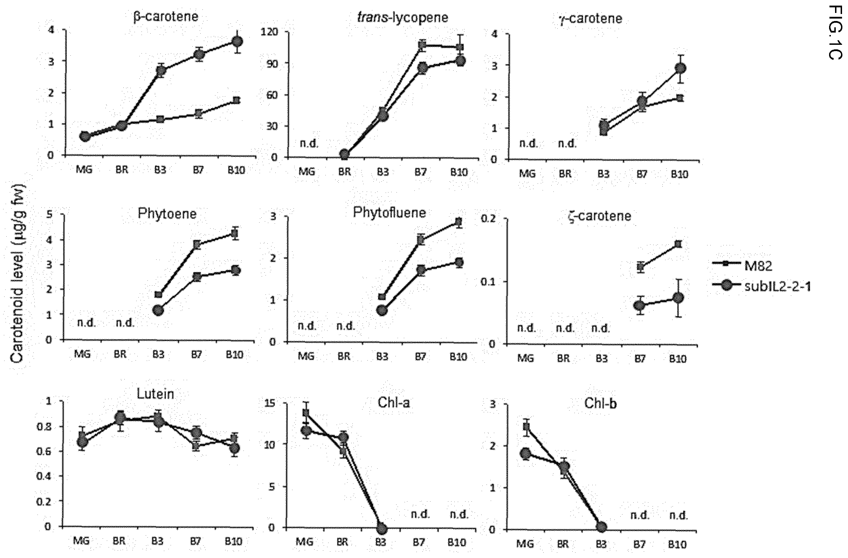

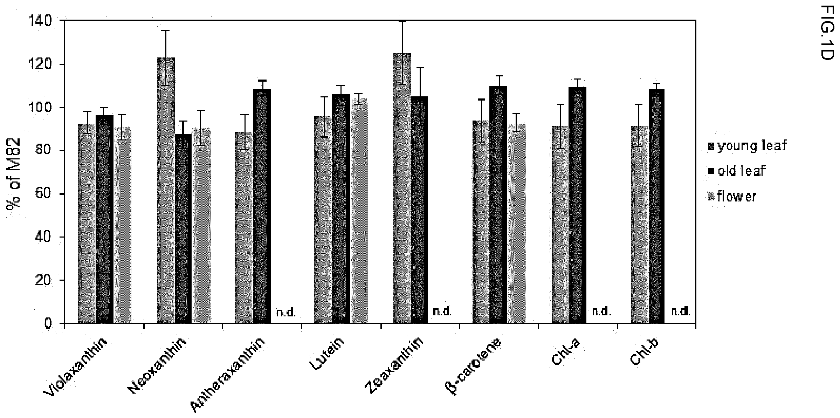

FIG. 1 is a diagram illustrating carotenoid profiles of IL2-1, IL2-2, subII,2-2-1, and M82: FIG. 1A. mature fruits of IL2-1, IL2-2, and M82; FIG. 1B. Relative levels of beta-carotene in IL2-1, IL2-2, and M82 cultivated under three different environmental conditions of trial 1 (outdoor, Florida, 2004), trial 2 (greenhouse, New York, 2010), and trial 3 (greenhouse, Daegu, 2016); (n = 5) FIG. 1C. Carotenoid and chlorophyll profiles of M82 and subIL2-2-1 (SubII,s containing overlapping introgressions of II,2-1 and IL2-2) during fruit ripening (n = 10). MG: mature green, BR: breaker (early ripening), B3: 3 days post breaker, B7: 7 days post breaker, B10: 10 days post breaker; FIG. 1D. Relative levels of carotenoids and chlorophyll in leaves and flowers of M82 and subIL2-2-1 (n = 5). n.d.: not detected.

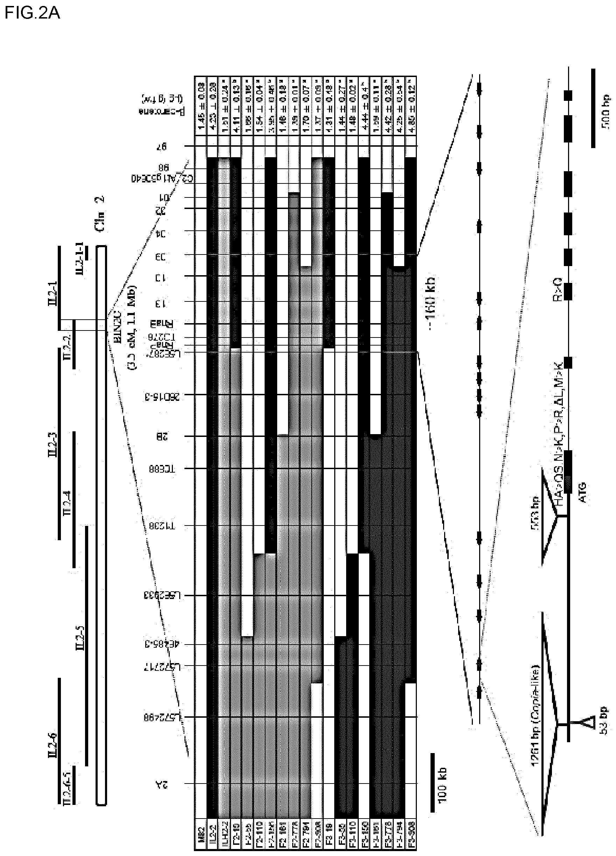

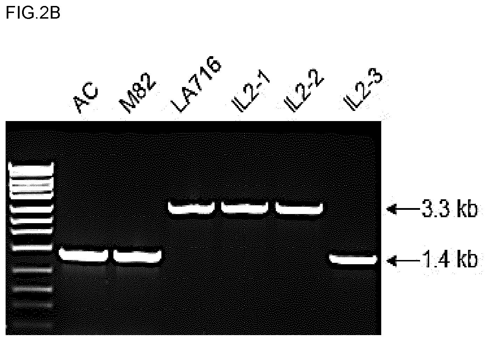

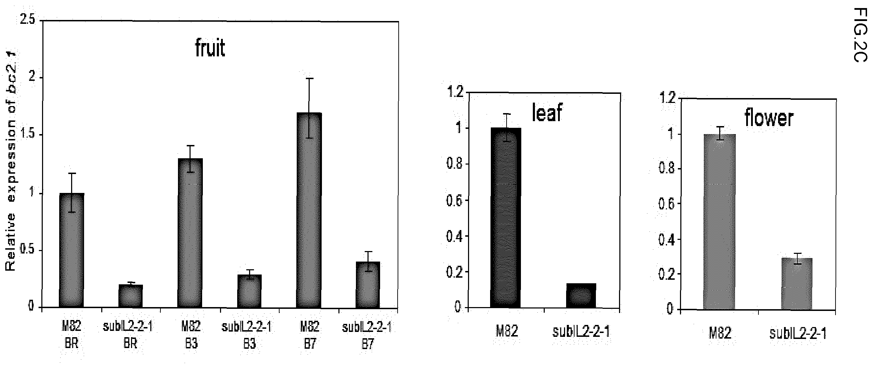

FIG. 2 is a diagram illustrating positional cloning of *bc2.1*: Top to bottom, IL gene map of chromosome 2, high-resolution genetic map derived from an IL2-2 F₂ population having beta-carotene level and statistical significance (n > 3, paired one tail t-test, *P* < 0.05), 15 ORFs (arrows) in a 160 kb region defined by markers between U582871 and 09, and gene model with two repeat element insertions in a promoter (triangle) and seven mutations in a coding region of an LA716 allele (compared with M82) (distinguished by orange arrows in 15 ORFs). A *Copia-*like retrotransposon (1261 bp) and a tomato-specific repeat element (553 bp) shown in a green triangle are inserted into the promoter of the LA716 *bc2.1* allele. An unknown fragment (53 bp) shown in an orange triangle is inserted into a *bc2.1* promoter of LA317 and LA1412. In a first exon, a red square represents a putative chromatophore target sequence and a black square represents eight exons. a = significantly higher than IL2-2, b = significantly higher than M82. FIG. 2B, Results of PCR analysis of the insertion of two repeat elements in a promoter region of *bc2.1* using cis1-F and cis1-R primers (Table 2). Gene expression analysis of *BC2.1*/*ORF2* in M82 and subIL2-2-1 in fruit, leaf and flower tissues (n = 5) at different developmental stages. Relative expression in fruits was normalized to an M82-BR stage.

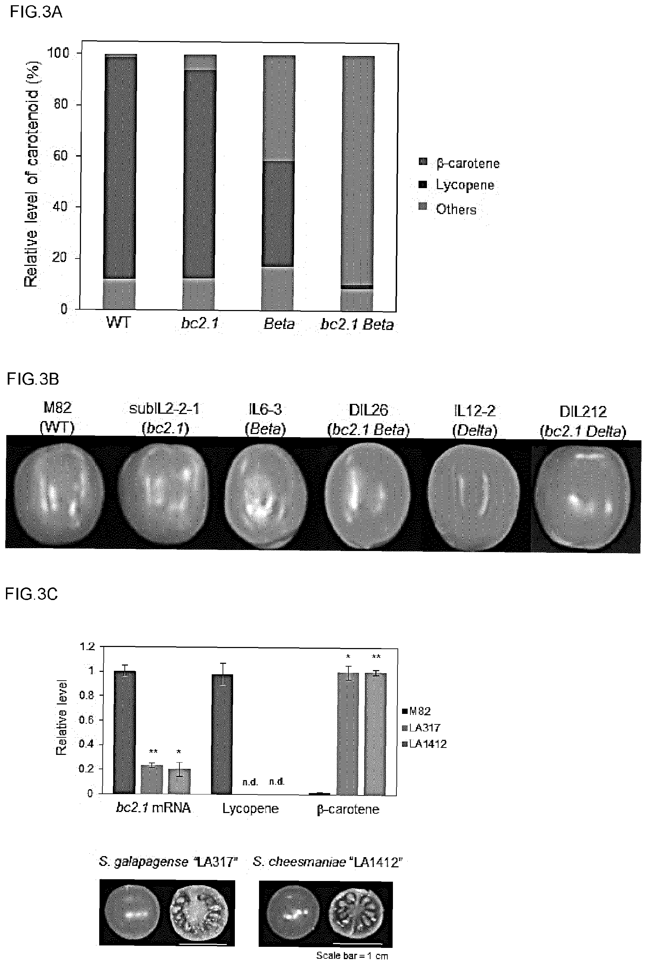

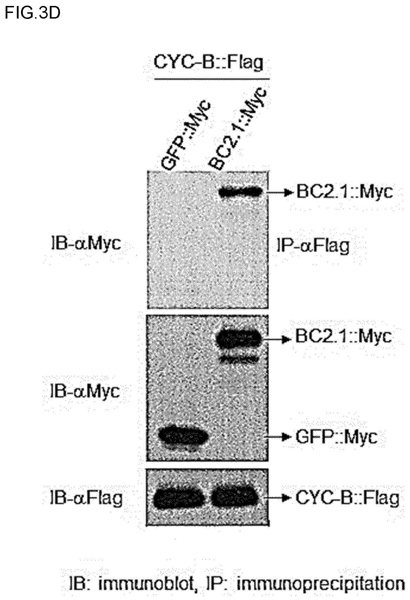

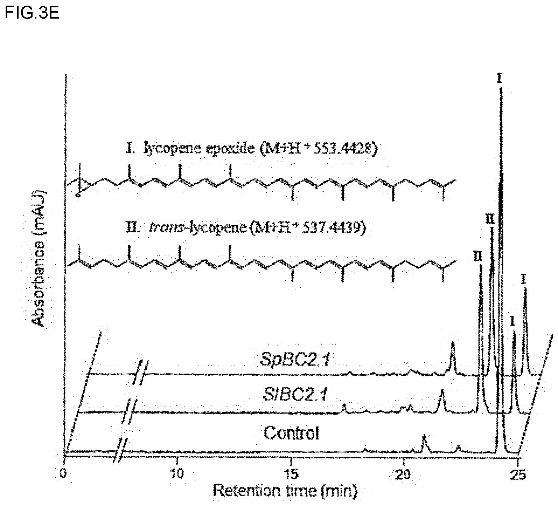

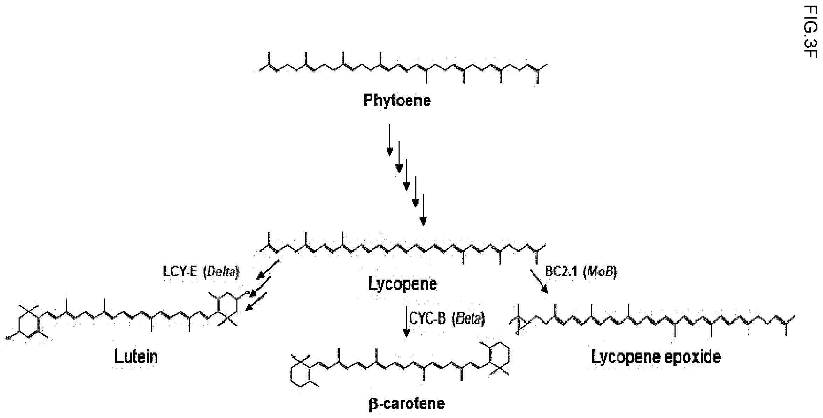

FIG. 3 illustrates the biochemical activity of genes encoded by *bc2.1* and BC2.1 alleles: FIG. 3A. HPLC analysis result of comparing relative carotenoid profiles in mature fruits of R*bc2*.*1* (subIL2-2-1), *Beta* (IL6-3), and *bc2.1 Beta* (DIL26) with wild type (M82); FIG. 3B. Mature fruits of M82 (WT), IL2-2-1 (*bc2.1*), IL6-3 (*Beta*), DIL26 (*bc2.1 Beta*), IL12-2 (*Delta*)*,* and DII,212 (*bc2.1 Delta*); FIG. 3C. Relative gene expression of *BC2.1*/*ORF2* and relative levels of lycopene and beta-carotene in *S. galapagense* "LA317" and *S. cheesmaniae* "LA1412" compared to M82 (n = 5). n.d.: not detected; FIG. 3D. BC2.1 and *CYC-B.* Immunoblot and co-immunoprecipitation analysis results showing relative association with Myc-BC2.1, *Myc-GFP,* and *CYC-B-Flag*; FIG. 3E. Functional analysis result of BC2.1/ORF2 protein in *E*. *coli,* which accumulates lycopene to produce lycopene epoxide. Peak identification: I. *trans-*lycopene; II. lycopene epoxide; FIG. 3F. New branch of carotenoid biosynthesis by *bc2.1. LCY-E*: lycopene e-cyclase, *CYC-B*: chromoplast specific β-cyclase, BC2.1: lycopene epoxidase, *MoB: Beta-modifier*
FIG. 4 illustrates results of genetically segmenting a *bc2.1* region using 100 F2-F3s from M82×IL2-2 and subIL2-1s. A blue dotted line indicates a candidate QTL region based on genotypes and phenotypes.
FIG. 5 illustrates results of quantitative RT-PCR analysis of carotenoid biosynthesis in mature fruits of M82 and subII,2-2-1 (n = 5).
FIG. 6 illustrates results of Pearson correlation analysis between beta-carotene and *bc2.1* expression in mature fruits of LA716 ILs.

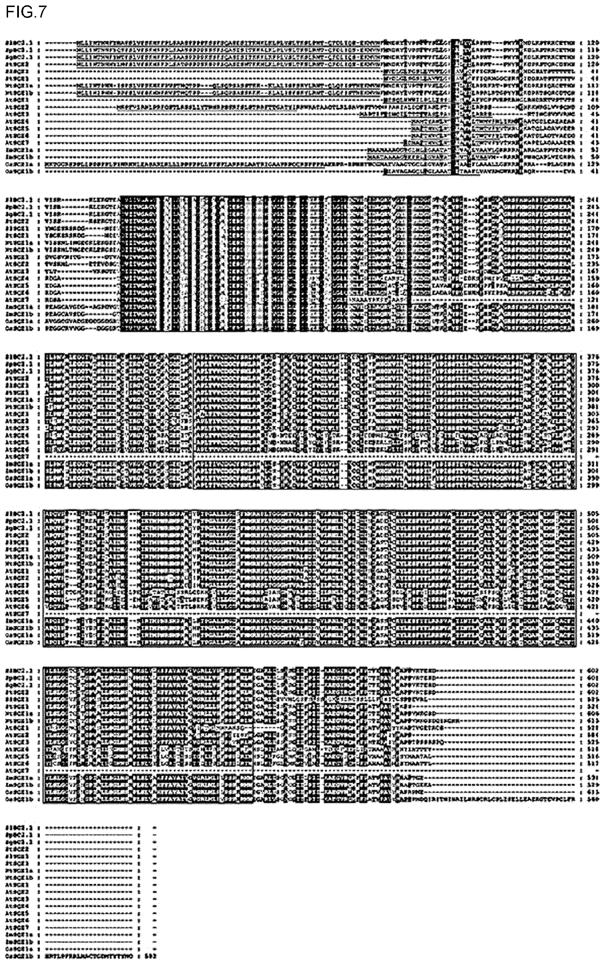

FIG. 7 illustrates multiple alignments of *bc2.1* analogues in plants. A red box represents an FAD binding domain, and a blue box represents a highly conserved region in a squalene epoxidase family. A green line represents a putative chromatophore target sequence based on the TargetP 1.1 server (http://www.cbs.dtu.dk/services/TargetP-1.1/index.php), an orange line represents a putative signal peptide and a yellow line represents a mitochondrial target sequence. Protein sequences used for analysis are as follows: S1BC2.1 (JX683513), SpBC2.1 (JX683514), SgBC2.1 (JX683515), StSQE2 (Sotub02g011310), S1SQE1 (Solyc04g077440), StSQE1 (XP_015168607), NtSQE1a (XP_016434789.1), NtSQE1b (XP_016502919.1), AtSQE1 (AT1G58440), AtSQE2 (AT2G22830), AtSQE3 (AT4G37760), AtSQE4 (AT5G24140), AtSQE5 (AT5G24150), AtSQE6 (AT5G24160), AtSQE7 (AT5G24155), ZmSQE1a (ZM09G25330), ZmSQE1b (ZM01G08660), OsSQE1a (Os03g12910), OsSQE1b (Os03g12900).
FIG. 8 illustrates a phylogenetic analysis result of BC2.1 analogues in plants and a neighbor-joining phylogenetic tree based on putative amino acid sequences of BC2.1 analogues in tomato, potato, tobacco, Arabidopsis thaliana, rice and corn.
FIG. 9 illustrates results of intracellular location analysis of BC2.1 in *N. benthamiana.* Scale bars = 5 µm.
FIGS. 10A to 10G illustrate alignments of *bc2.1* promoter sequences in M82, LA317, LA1412 and LA716. Major insertions in wild species are indicated by bold and colored nucleotides. Start codons are underlined. Red: *Copia*-like retrotransposon in LA716, Green: Tomato-specific repeat element in LA716, Blue: Unknown 52 bp nucleotides in LA317 and LA1412.
FIG. 11 illustrates results of qRT-PCR analysis showing transcript levels of *bc2.1* in wild-type and T₀RNAi-transgenic tomatoes of mature fruits (B+10). Relative transcript levels for wild type (M82 or AC) were shown.

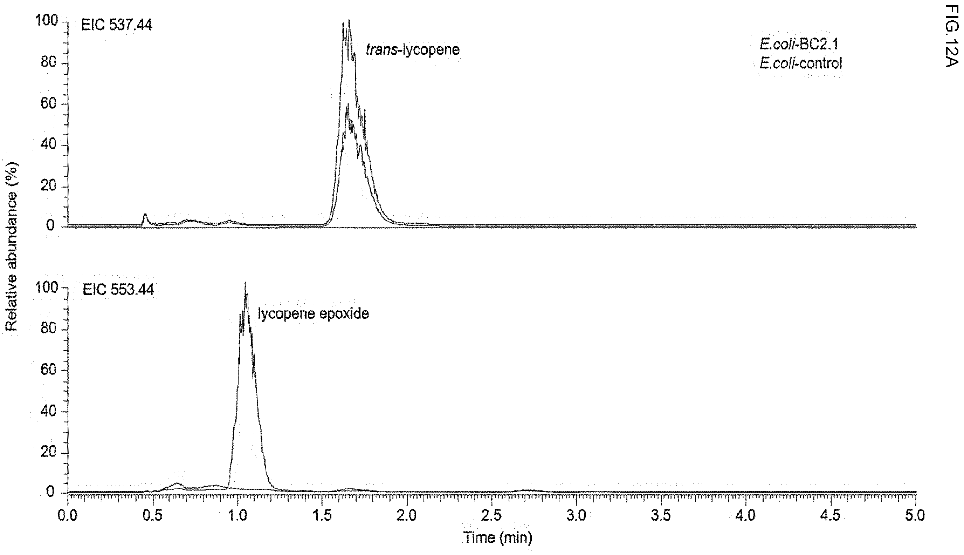

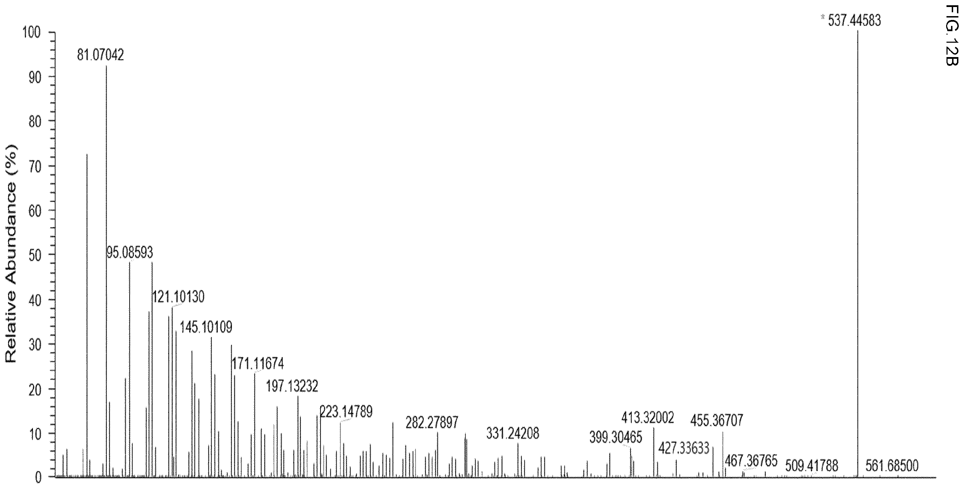

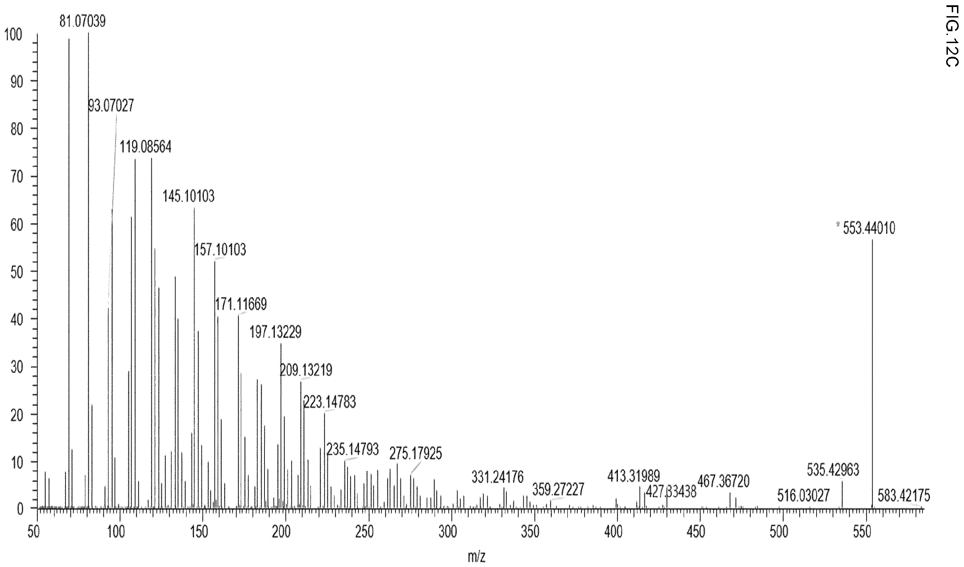

FIG. 12 illustrates LC-MS/MS characterization of BC2.1 activity assay using LC-APCI-MS/MS: FIG. 12A illustrates an overlay of extracted ion chromatograms (EIC) from *trans-*lycopene (extract from *E. coli* transformed with empty vector, green) and lycopene epoxide (extract from BC2.1 expressing *E. coli* cells, red) (FIG. 3E). An upper panel shows EICs of m/z 537.44 Da [M+H]⁺ from a control (ppm error: 0.12) and a lower panel shows m/z 553.44 Da [M+H]⁺ (ppm error: 0.23). b. MS-MS segmentation spectrum at m/z 537.44, *trans*-lycopene; c. MS-MS segmentation spectrum at m/z 553.44, lycopene epoxide.
FIG. 13 illustrates LC-MS/MS characterization of BC2.1 activity assay using UPLC-ESI-QTOF-MS/MS: FIG. 13A illustrates UV-Vis spectra of *trans*-lycopene and lycopene epoxide extracted at 471 nm. FIG. 13B illustrates an MS spectrum of lycopene epoxide. FIG. 13C illustrates an MS/MS spectrum of lycopene epoxide.
FIG. 14 illustrates lycopene epoxide contents of wild-type (M82 and AC), subIL2-2-1 and RNAi transgenic tomatoes in mature fruits (10 days post-breaker, n = 5).
FIG. 15 illustrates subIL2-2-1 introgression location and subIL2-2-1 breeding tree.

### [Modes of the Invention]

Hereinafter, the present invention will be described in more detail through Examples. However, these Examples are more specifically illustrative the present invention, and the scope of the present invention is not limited to these Examples.

### Plant materials

To evaluate introgression line (IL) fruit carotenoids and their environmental changes, tomatoes grown outdoors in Florida (spring, 2004), grown in a greenhouse in New York (fall, 2010) and outdoors in Daegu (spring, 2004) were used. Tomato mapping populations and transgenic plants were grown under standard conditions (27/19°C; 16/8 h light/dark) in a greenhouse of the Boyce Thompson Institute for Plant Research (NY, USA). Seeds were obtained from the Tomato Genetics Resource Center at the University of California, Davis (CA, USA) (http://tgrc.ucdavis.edu/) and Hebrew University of Israel (Jerusalem, Israel).

### Accession codes

Sequences were obtained from GenBank: *bc2.1* (JX683513), *Spbc2.1* (JX683514), and *Sgbc2.1* (JX683515).

### Positional cloning of bc2.1

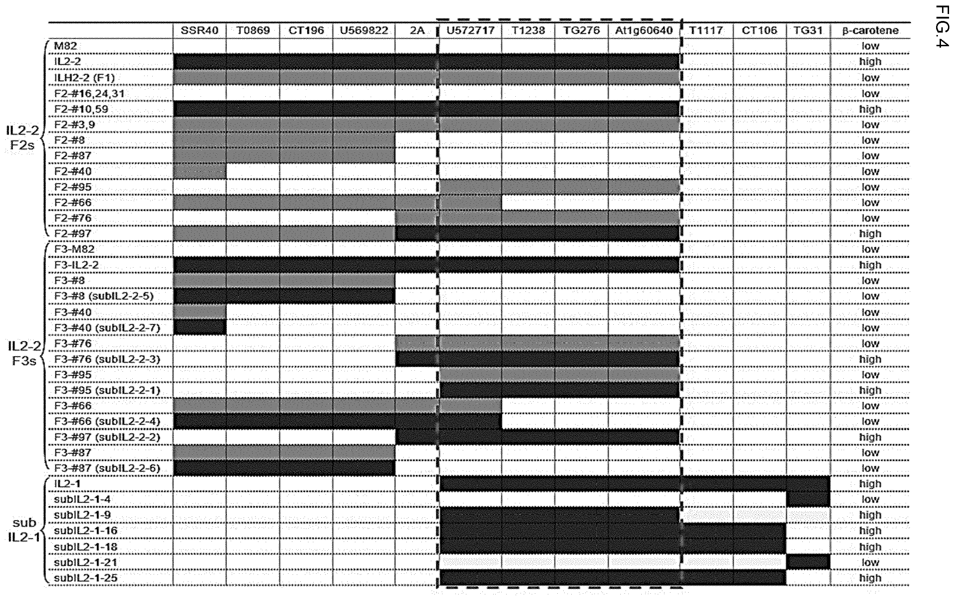

Genetic analysis of *bc2.1* was performed by using 100 plants of an M82×IL2-2 F₂ population in addition to lines derived from IL2-1 representing a subset of a 2-1 genetic introgression line (also derived from M82×IL2-1 F₂) acquired from the Hebrew University of Israel (FIG. 4). It was confirmed using the plant materials that *bc2.1* was located near the top of chromosome 2 between DNA markers 2A and C2_At1g60640 (FIG. 4), corresponding to a 1.1-Mb segment of tomato chromosome 2 (covering a 29.9-31.0-Mb region as defined in tomato genome assembly SL2.40).

Additional high-resolution mapping of *bc2.1* was performed using 1100 F₂ plants derived from M82×IL2-2 crossing. *Bc2.1* flanking markers 2A and 98 (the latter was a PCR marker upstream of IL2-2 defined in an initial small mapping population) were used for recombinant screening. Eight F₂ recombinants were obtained in the 1.1-Mb region and beta-carotene was assayed from mature fruits at the same developmental stage labeled by HPLC in their F₃ progeny which was the same type as a *bc2.1* LA716 or M82 allele (paired one-tailed *t*-test, *P* < 0.05). Between markers 09 and U582871 (FIG. 2A), a 160-kb genetic introgression fragment derived from LA716 was co-segregated with beta-carotene, and this fragment contained 15 predicted ORFs. Markers used for genotyping were shown in Table 1. Sequencing of candidate *bc2.1* alleles from M82 and subIL2-2-1 was performed and gene expression was evaluated by qRT-PCR. For carotenoid analysis, fruits were harvested from all genotypes by developmental stage (7 days post-breaker). Pericarp tissues were directly frozen in liquid nitrogen and stored at - 80°C. The tissues were crushed using liquid nitrogen for carotenoids and RNA extraction.

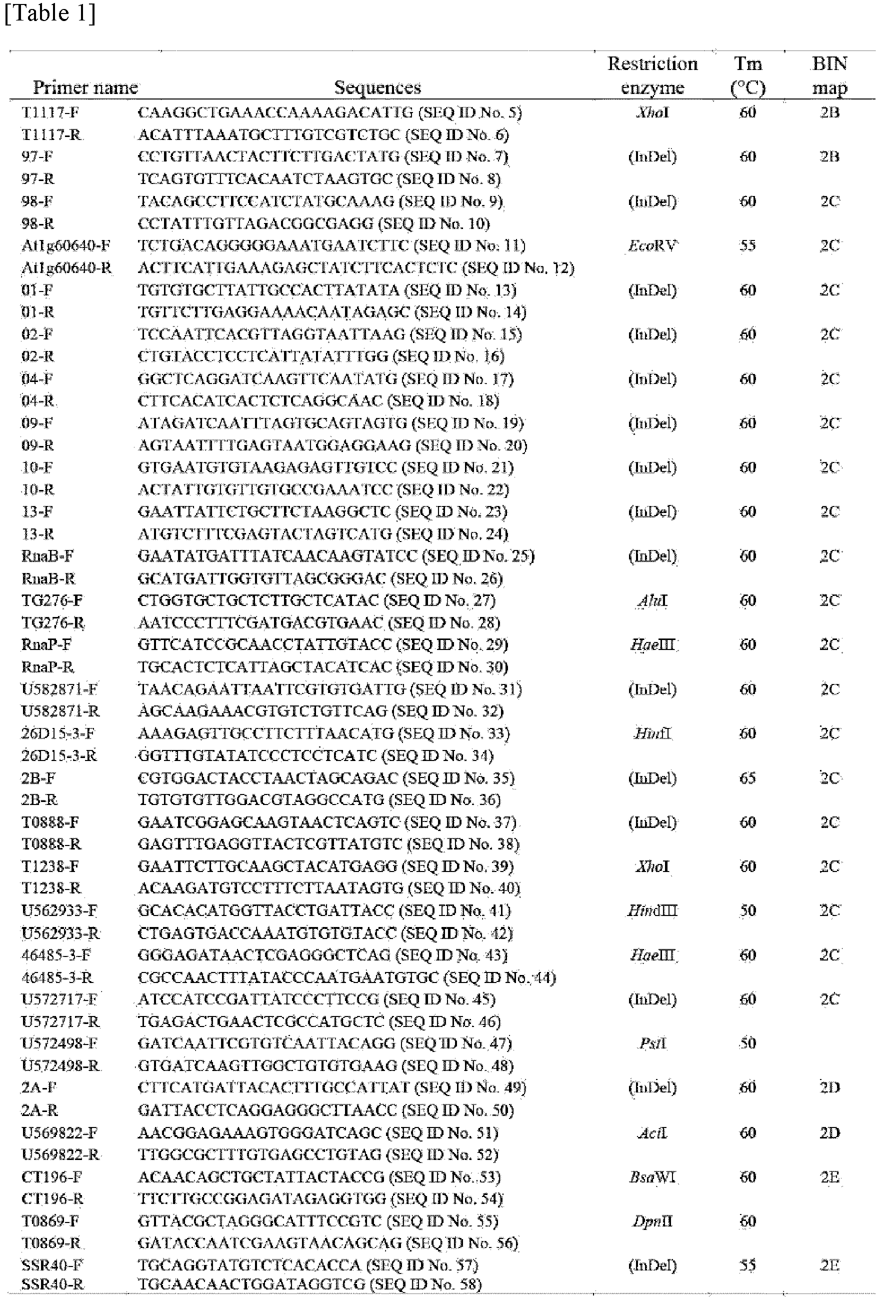

### Carotenoid profiling by HPLC

About 100 mg of frozen and crushed pericarp from individual fruits (including biological replicates) were used for carotenoid extraction. All results from each experiment were compared with control fruit genotypes grown under the same conditions. Carotenoids and chlorophyll were extracted and quantified by HPLC.

### Development of RNAi construct and tomato transgenics

A *bc2*.*1*-specific 282-bp DNA fragment (EST clone: TUS-64-H10) located in a 3'-UTR region of cDNA (i.e., bases 1866 to 2147 of GenBank accession number JX683513) was amplified by PCR using Phusion High-Fidelity DNA polymerase (New England Biolabs, MA, USA), gene-specific primers, RNAi-F and RNAi-R (Table 2), and EST clone as template. The purified cDNA fragment was cloned into pHellsgate 2 as an inverted repeat sequence under the control of a 35S promoter using a Gateway^{™} cloning system (Gateway^{™} BP Clonase^{™} Enzyme Mix, Invitrogen, CA, USA). The integrity of the structure was confirmed by sequencing, and introduced into M82 and AC tomato species using *Agrobacteriumtumefaciens* LBA4404 by a previously published method (Fillatti, J.J. et al., Efficient Transfer of a Glyphosate Tolerance Gene into Tomato Using a Binary Agrobacterium-Tumefaciens Vector. Bio-Technology 5, 726-730 (1987)). Transgenic plants were confirmed by PCR using a CaMV-35S-specific primers, 35S-F and 35S-R (Table 2), and subjected to Southern blot analysis using a CaMV-35S-specific probe.

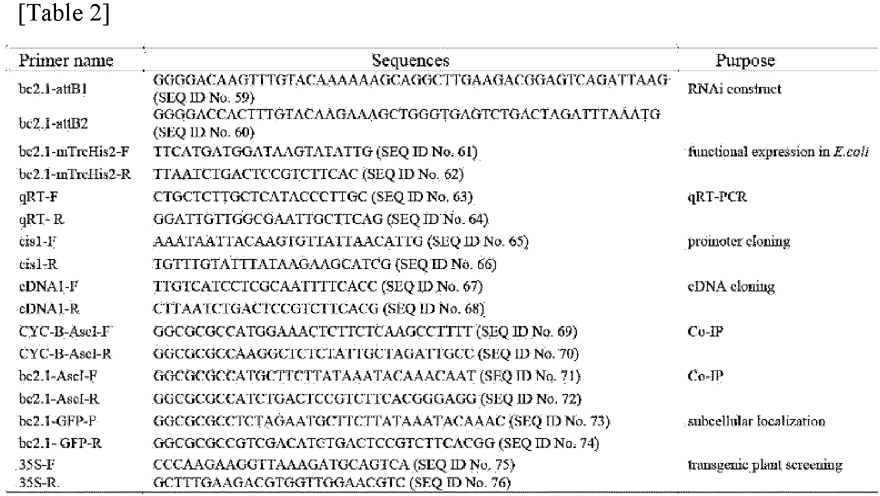

### Measurement of mRNA accumulation by qRT-PCR

Total RNA (2 µg) was reverse transcribed with random hexamers and Superscript III (Invitrogen). Purified cDNA (2 ng) was used as a template for qRT-PCR. qRT-PCR analysis was performed with gene-specific primers (Tables 2 and 3) using an ABI PRISM 7900HT (Applied Biosystems, CA, USA) real-time thermocycler and a SYBR Green PCR master mix (Applied Biosystems).

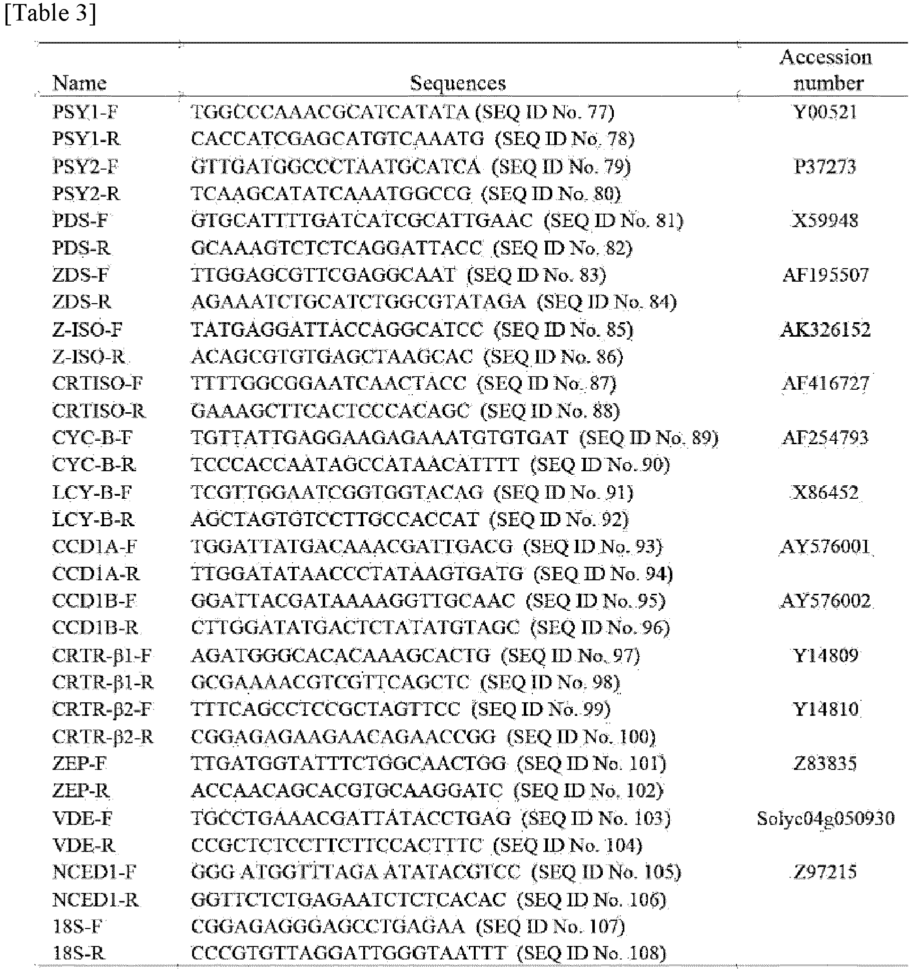

### Subcellular localization of BC2.1

pCAMBIA2300-eGFP for carrying increased GFP under the control of a CaMV 35S promoter, was used for subcellular localization prediction analysis. Whole *bc2.1* excluding a stop codon was amplified using gene-specific primers BC2.1-GFP-F and BC2.1-GFP-R. The amplified *bc2.1* and pCAMBIA2300-eGFP were double-cleaved with *Xba*I and *Sal*I at 37°C for 4 hours, and then *bc2.1* was ligated to a compatible *Xba*I/*Sal*I site of pCAMBIA2300-eGFP using T4 DNA ligase (Promega Corporation, WI, USA). The resulting plasmid pCAMBIA2300-BC2.1-eGFP was transferred to *E. coli* DH5α and sequenced. Appropriate plasmids were transformed into *Agrobacterium tumefaciens* GV3101 by a freeze-thaw method. *Agrobacterium* was incubated at 28°C until OD 600 was 0.7, resuspended in an induction buffer (10 mM MES, 10 mM MgCl₂, 200 µM acetosyringone), and then incubated at room temperature for 2 hours before agroinfiltration. Young leaves of 6-week-old *N. benthamiana* plants were agroinfiltrated with GV3101 containing either pCAMBIA2300-eGFP or BC2.1-eGFP. The intracellular localization of a GFP fusion protein was observed by confocal laser-scanning microscopy (LSM700, Carl Zeiss, Oberkochen, Germany). Chloroplasts were identified by red auto-fluorescence at 555 nm and green fluorescence (GFP) at 488 nm.

### Development of double genetic introgression lines

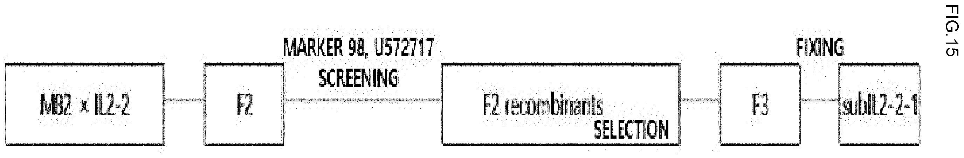

To identify a *bc2.1* locus, an F₂ population obtained by hybridizing M82 and IL2-2 was constructed, and located between marker 98 and U572717 (Table 1) on chromosome 2 to breed subIL2-2-1 with an increased β-carotene content. The subIL2-2-1 introgression location and subIL2-2-1 breeding tree were illustrated in FIG. 15.

IL6-3 and IL12-2 were obtained from the Tomato Genetics Resource Center at the University of California, Davis (CA, USA) (http://tgrc.ucdavis.edu/).

For separation of a double genetic introgression line DIL26, subIL2-2-1 was crossed with IL6-3. Among the resulting 75 F₂ plants, homozygous lines containing double mutations were obtained using gene-specific PCR markers of *bc2.1* (Table 1) and *CYC-B.* One line, DIL26, was used for further analysis. For separation of a genetic introgression line DIL212, subIL2-2-1 was crossed with IL12-2. Among the resulting 82 F₂ plants, homozygous lines containing double mutations were obtained using gene-specific PCR markers of *bc2.1* (Table 1) and *LYC-E.* One line, DIL212, was used for further analysis. Carotenoids were analyzed by HPLC in mature fruits of M82, subEL2-2-1, IL6-3, IL12-2, DIL26 and DII,212 (Table 4).

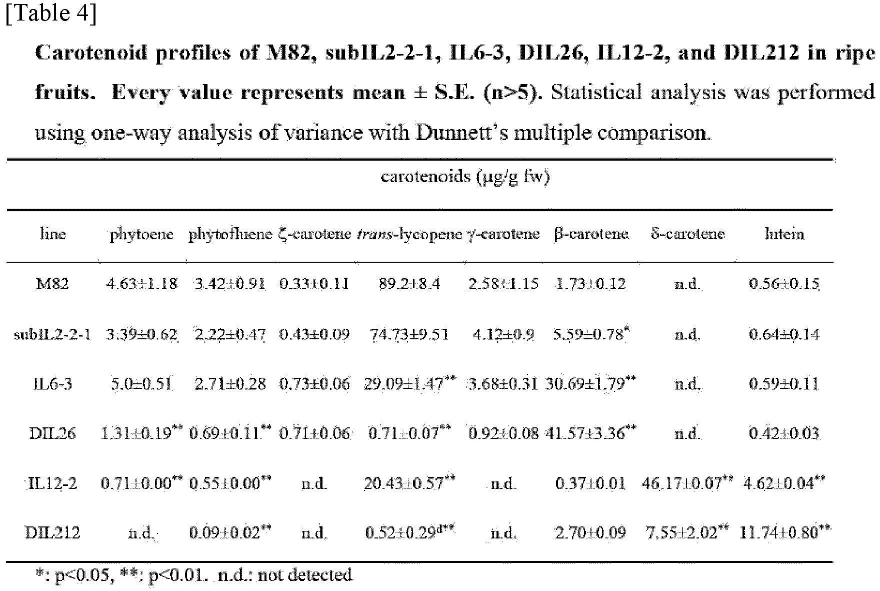

### DNA construct for Asrobacterium-mediated transient expression and co-immunoprecipitation in N. benthamiana

*Agrobacterium-*mediated transient expression in *N. benthamiana,* co-immunoprecipitation and immunoblot were performed. Plant overexpression constructs for Myc-GFP were previously published and used (Bhattacharjee, S. et al. Virus resistance induced by NB-LRR proteins involves Argonaute4-dependent translational control. Plant J. 58, 940-951 (2009)). *CYC-B* and BC2.1 with an AscI site at each end were amplified with oligonucleotides (CYC-B-AscI-F + CYC-B-AscI-R and BC2.1-AscI-F + BC2.1-AscI-R, respectively, Table 2) from fruit cDNA. The amplified *CYC-B* and BC2.1 fragments were ligated to pER8-Flag and pER8-Myc, respectively, to generate pER-*CYC*-*B*-Flag and pER-BC2.1-Myc.

### Expression of BC2.1 in trans-lycopene-accumulating E. coli

PCR fragments were amplified using the *BC2.1*/*ORF2* full cDNA of M82 and subIL2-2-1 and cloned into a pTrcHis2-TOPO vector (Invitrogen, CA, USA) using an mTrcHis2 primer (Table 2), and confirmed by sequencing. pAC-LYC was co-transformed into *E. coli* strain BL21-AI with the pTrcHis2-TOPO-BC2.1 construct. A control consisting of a *trans*-lycopene-producing strain and pTrcHis2-TOPO without BC2.1 was subjected to the same induction treatment. 3 mL of the culture incubated overnight was inoculated into 50 mL of an LB medium containing appropriate antibiotics and 0.2% glucose. The culture was grown at 28°C until the absorbance at 600 nm was 0.5. The expression of the protein was induced by adding 1 mM IPTG and incubated overnight at 28°C in the dark. Then, an equal volume of acetone was added and then a double volume of ethyl acetate was added. Phases were separated by adding water and an ethyl acetate phase was stored for HPLC and LC-MS/MS analysis. After centrifugation, the medium from the culture was split twice with an equal volume of ethyl acetate. HPLC fractions were dried and resuspended in ethyl acetate, and then used in HPLC as described above.

### LC-MS/MS analysis of carotenoids for enzyme assay

LC-MS/MS analysis was performed by Mass Spectrometry Convergence Research Center (Kyungpook National University, Daegu, South Korea). A 5 µl extract from the enzyme assay (FIG. 3E) was analyzed using an Ultimate 3000 U-HPLC system (Thermo Fisher Scientific, MA, USA) and a Q EXACTIVE^{™} Focus massspectrometry system (ThermoFisherScientific) using atmospheric pressure chemical ionization (APCI) (positive mode). Chromatographic separation was performed using a Kinetex C18 column (100 × 2.1 mm; Phenomenex, Torrance, CA). A mobile phase was run with 0.1% ammonium acetate-containing methanol (A) and methyl *tert*-butyl ether (B) at a flow velocity of 0.5 mL/min under conditions of 90% A and 10% B at 25°C for 5 minutes. The APCI parameters were as follows: sheath gas flow rate, 50 arbitrary units; auxiliary gas unit flow rate, 12 arbitrary units; vaporizer temperature, 300°C ; capillary temperature, 320°C; discharge current, 5 mA; S-lens radiofrequency level, 95; resolving power, 70,000. For a target compound, a scan range of 520 to 620 m/z was selected. An automatic gain control was set to 1 × 10⁶ and the injection time was set to 100 ms.

In addition, UPLC-ESI-QTOF-MS/MS was performed on a high-resolution 6540 QTOF-MS/MS spectrometer (Agilent, Santa Clara, CA, USA) with electrospray ionization (ESI, positive mode) in the detection range of 500 to 600 m/z. LC separation was performed on an Agilent 1290 UPLC system (Agilent) as previously published. UPLC-ESI-QTOF-MS/MS was performed by Metabolomic Discoveries GmbH (Potsdam, Germany).

### Quantification of squalene and 2,3-oxidosqualene

For extraction and profiling of squalene and 2,3-oxidosqualene, about 200 mg of pericarp was used at 7 days after fruit breaker. The extraction of squalene and 2,3-oxidosqualene was performed by a known method (Khachik, F. et al. Chemistry, distribution, and metabolism of tomato carotenoids and their impact on human health. Exp Biol Med (Maywood) 227, 845-851 (2002)), and quantitative analysis was performed using 6540 Q-TOF/LC-MS (Agilent, CA, USA) and reverse-phase C18 columns (100 × 3 mm, 1.8 mm, Agilent). Standard squalene and 2,3-oxidosqualene were purchased from Sigma-Aldrich (St. Louis, MO, USA).

### Results

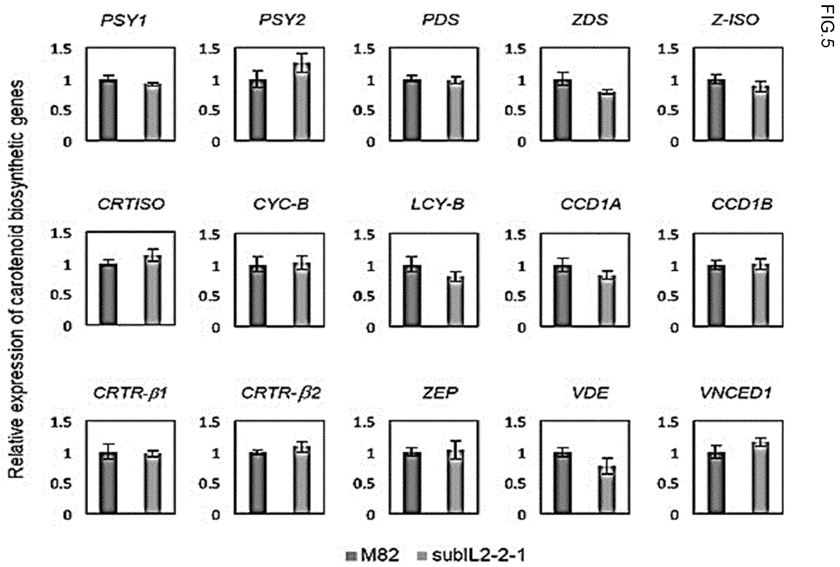

To identify natural variations affecting carotenoids in tomato, the carotenoid content of mature fruits was evaluated in genetic introgression lines (ILs) of *S. pennellii* "LA716". Among the ILs, IL2-1 and IL2-2 (FIG. 1A) had overlapping portions of introgression chromosome and the fruit color and shape were not visually distinguished from a control *S*. *lycopersicum* "M82", but the content of heritable beta-carotene was 2.5 times higher therethan (FIG. 1B). In the present invention, this beta-carotene quantitative trait locus (QTL) was designed and named *bc2.1.* HPLC carotenoid profiling was performed in subIL2-2-1 to study additional effects of the *bc2.1* locus. SubIL2-2-1 was grown in the F3 population of M82×IL2-2 (FIG. 4) to contain a region overlapping LA716 segments of IL2-1 and IL2-2 (referred to as BIN2C). Compared to M82, subIL2-2-1 did not show significant mutations in carotenoids or chlorophyll in leaf and flower tissues (FIG. 1D). Interestingly, beta-carotene increased only during fruit ripening, and beta-carotene precursors such as phytoene, phytofluene, and *trans-*lycopene slightly decreased compared to M82 (FIG. 1C). The *bc2.1* did not significantly change the transcriptional accumulation of carotenoid biosynthetic genes, and it was suggested that the quantitative change in beta-carotene accumulation in subIL2-2-1 tomato fruit was clearly caused by a new alternative means rather than a change in mRNA accumulation of a previously known synthetic gene (FIG. 5). In addition, among genes related to carotenoid accumulation in plants, a gene located in BIN2C was unknown.

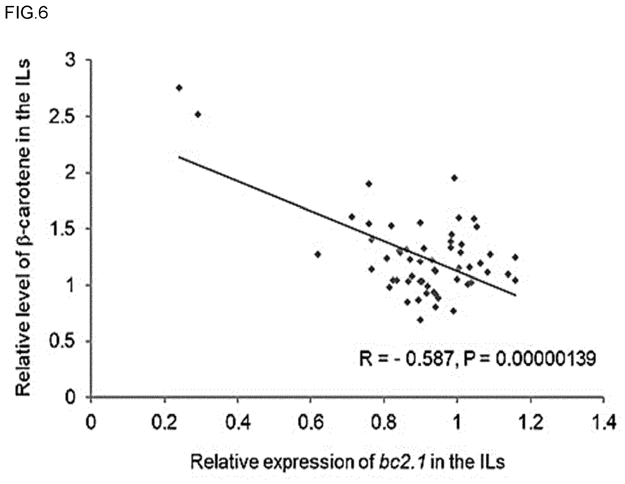

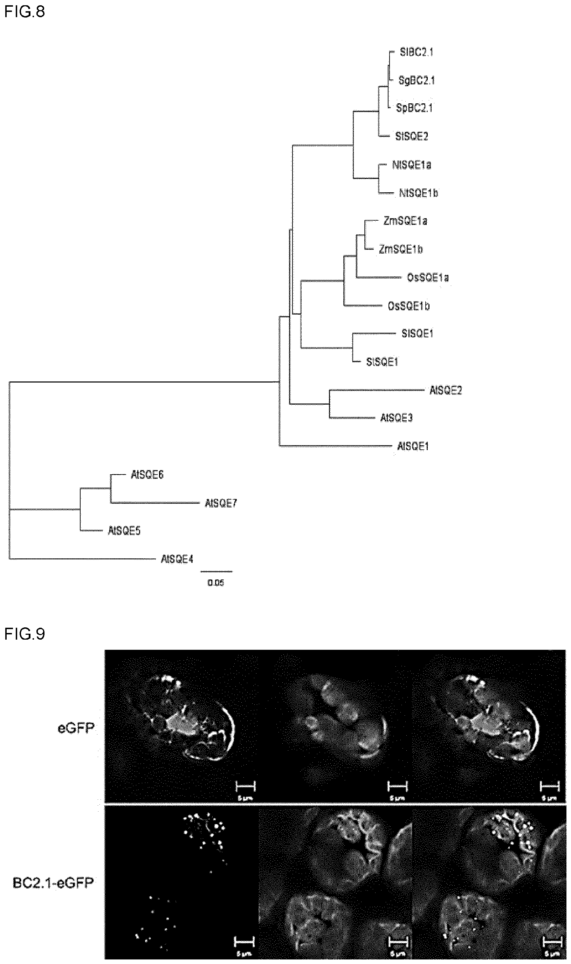

High-resolution mapping of *bc2.1* was performed using the M82×IL2-2 F₂ population and subIL2-1s (FIG. 2A). A segment introgressed with LA716-derived chromosome 160-kb between markers 09 and U582871 was separated with the increased beta-carotene content and contained 15 open reading frames (ORFs) (FIG. 2A). It was confirmed that a *bc2.1* allele, which was a cause of beta-carotene enhancement, was inherited recessively through an F₁ phenotype, and in the case of heterozygous plants, a phenotype similar to that of plants homozygously having an M82 allele was shown (FIGS. 2 and 4). These results suggested that the increased content of beta-carotene in ILs was caused by a loss-of-function allele derived from LA716. From RNA-seq from LA716 (FIG. 6) and analysis of genomic DNA sequences, ORF2 which was induced during fruit ripening and highly homologous to squalene epoxidase (SQE: FIG. 7) was identified as a candidate gene. In plants, SQE family members include 7 genes from Arabidopsis and 2 genes from rice, corn and solanaceae plants (FIG. 8).

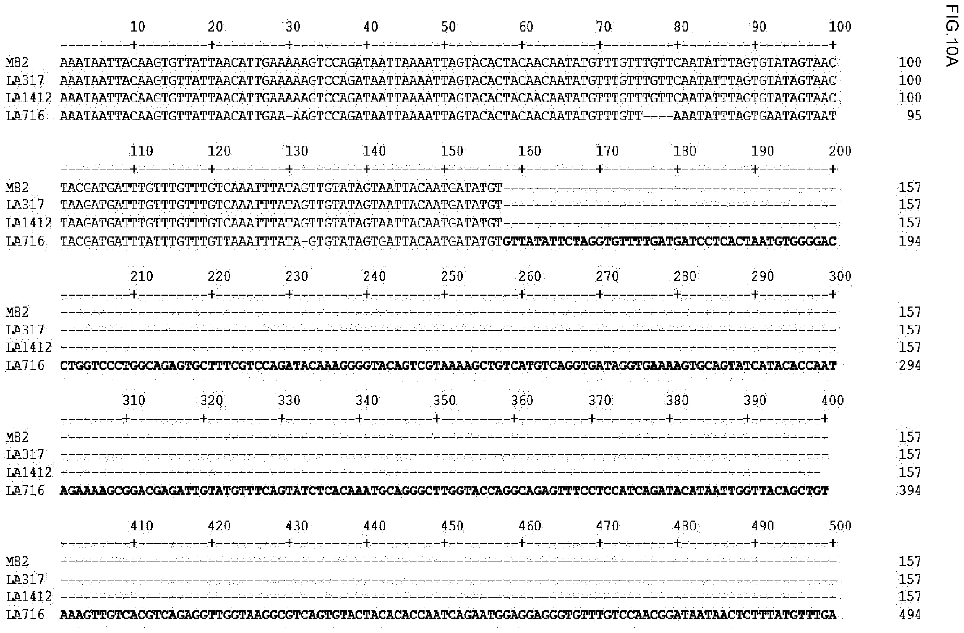

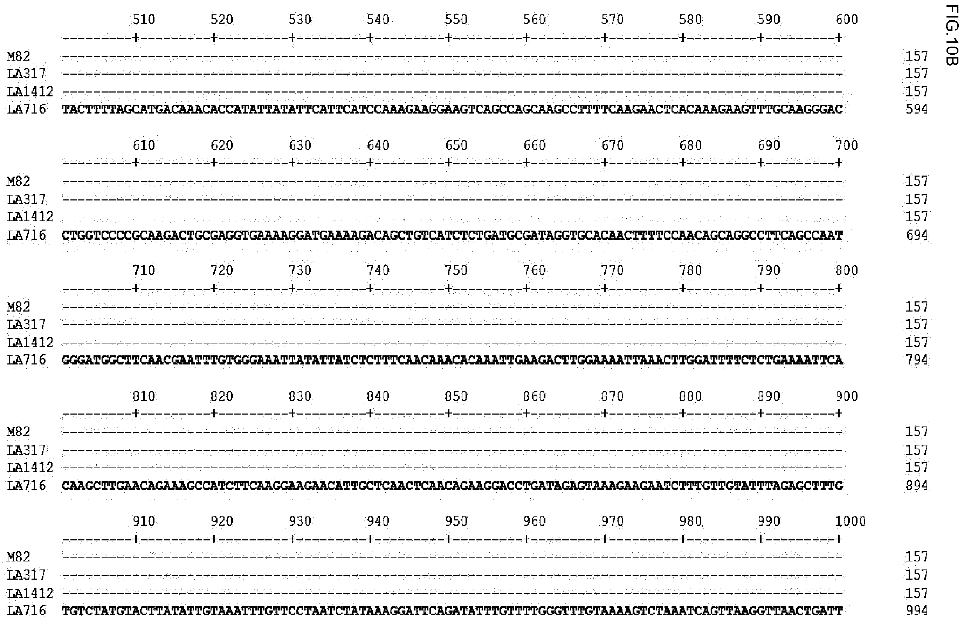

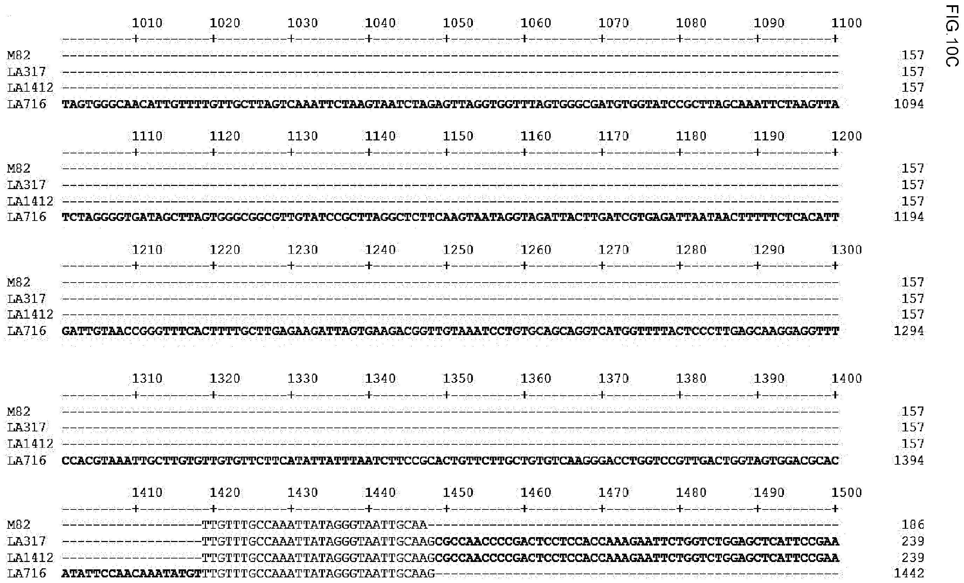

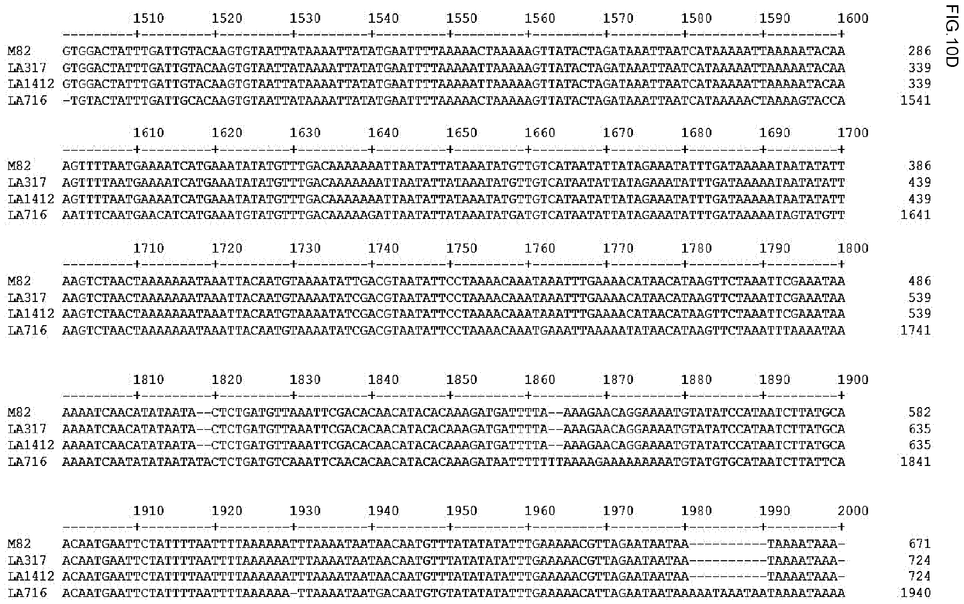

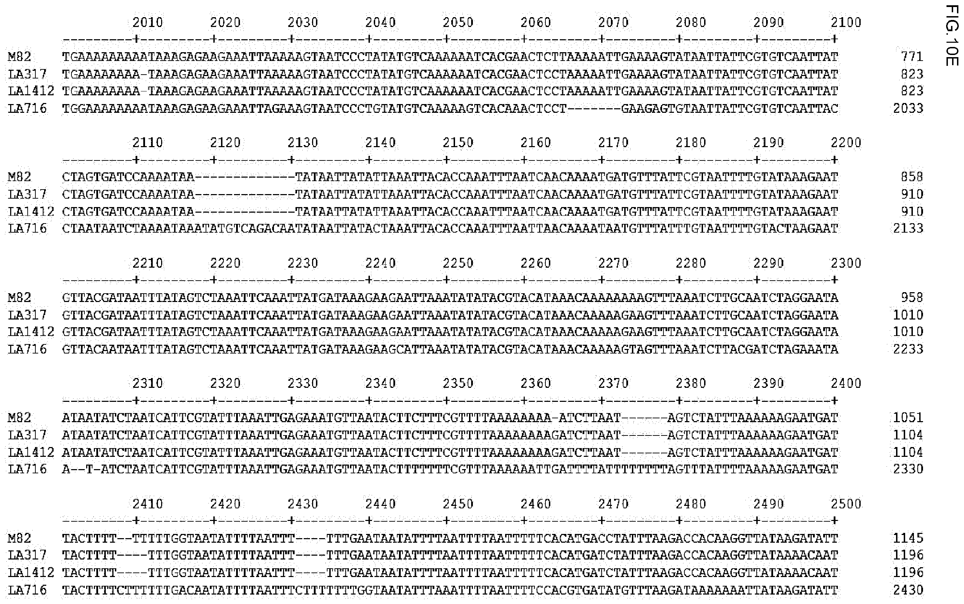

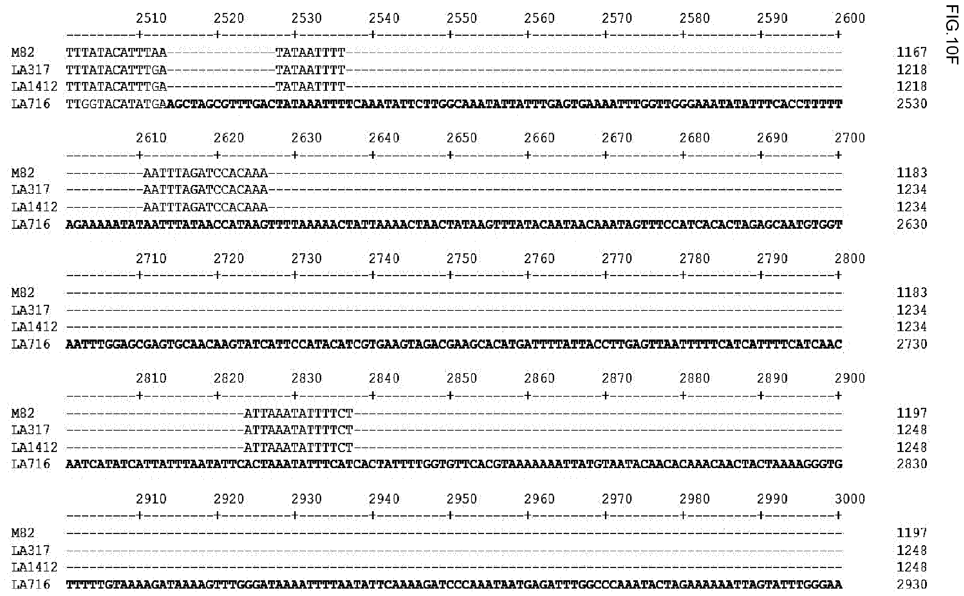

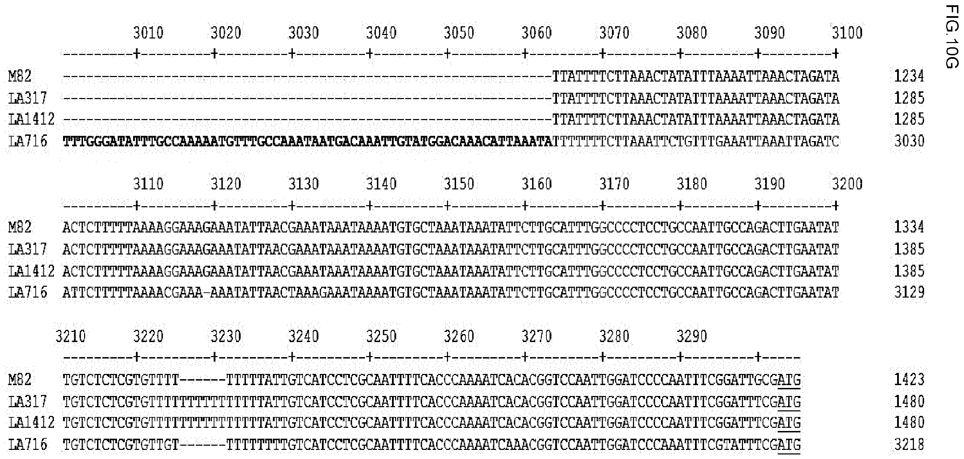

The expression of *BC2.1*/*ORF2* was significantly decreased compared to M82 not only during leaf and flower ripening but also during fruit ripening (FIG. 2C), which further supported that this gene was a cause of a *bc2.1* fruit beta-carotene phenotype. In a putative promoter sequence 1.4 kb upstream of ORF2, an insertion of a 1.8 kb sequence was identified in the LA716 allele. When compared to the M82 allele, a *Copia*-like retrotransposon and a tomato-specific repeat element were inserted into LA716 at positions 1263 and 223 bp upstream from an initiation codon, respectively (FIGS. 2B and 10), which may cause a decrease in ORF2 expression in subIL2-2-1 (FIG. 2C). In *S. pennellii,* a *Copia* element located in a proximal promoter to the gene leaded to lower expression of these genes than in orthologous genes in *S*. *lycopersicum* without the *Copia* element. In addition, six amino acid substitutions and one amino acid deletion were identified in a coding region of LA716 (FIGS. 2A and 7). As described below, mutations in the coding sequence of an allele did not affect its activity (FIG. 3E), and it was suggested that any differences in phenotype resulted from a decrease in gene expression.

SQE promoted the metabolism of squalene to produce 2,3-oxidosqualene, which was a precursor to cyclic triterpenoids of all angiosperms. Carotenoid metabolizing enzymes were mostly classified as being present in chromatophores, and the metabolism of triterpenoids, including squalene, mainly remained in the ER and cytosol. Unlike Arabidopsis SQE family members, ORF2 contained a predicted chromatophore-targeting peptide in the N-terminal region (FIG. 6). To confirm whether reduced expression of ORF2 in subIL2-2-1 affected a squalene metabolism, squalene and 2,3-oxidosqualene were quantified by Q-TOF-LC/MS (Table 5). Squalene was not detected in either M82 or subII,2-2-1 mature fruits, and the levels of 2,3-oxidosqualene were very similar between the two species.

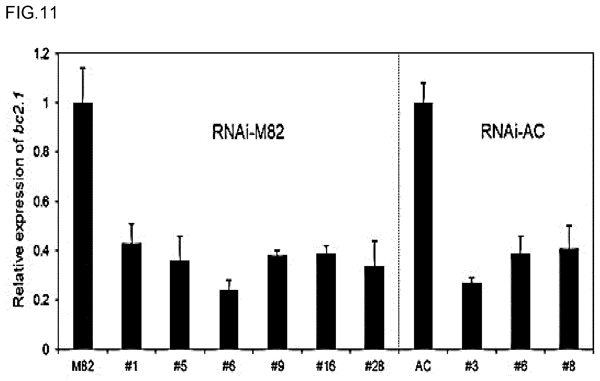

RNAi experiments were conducted to enhance the beta-carotene content of tomato fruits by ORF2, as a mediator of *bc2.1.* The RNAi structure of ORF2 was introduced into M82, which had a relatively low beta-carotene content (1.5 to 2.0 µg/g fw) in fruit. The same construct was also transformed into Ailsa Craig (AC), which had a relatively high beta-carotene content (about 14 µg/g fw) in mature fruits. Six independent transgenic lines in M82 and three independent transgenic lines in AC were generated (Table 6) and showed increased beta-carotene and reduced ORF2 expression (FIG. 11). In all nine RNAi lines, the beta-carotene content increased 2 to 4 times, and other carotenoids had no significant change in the RNAi lines (Table 6). In an M82-RNAi line, the carotenoid phenotype observed in a T0 generation was maintained in a T1 generation (Table 6). In summary, the results confirmed that a decrease in *ORF2* expression increased beta-carotene concentration in mature fruits, and decreased expression of the LA716 allele of this gene was a fundamental determinant of the bc2.1 QTL. These results also demonstrated that *BC2.1*/*ORF2* had a negative effect on b-beta-carotene accumulation in mature tomatoes.

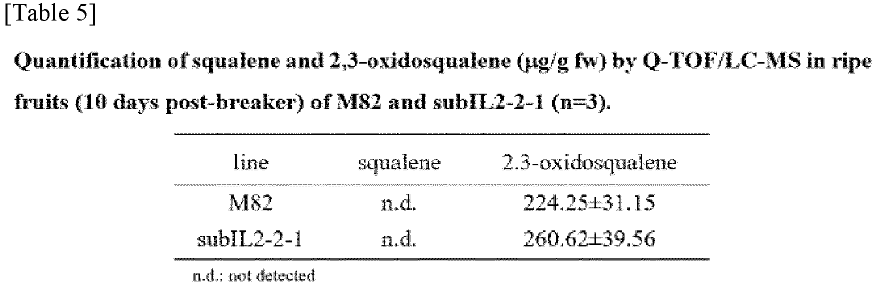

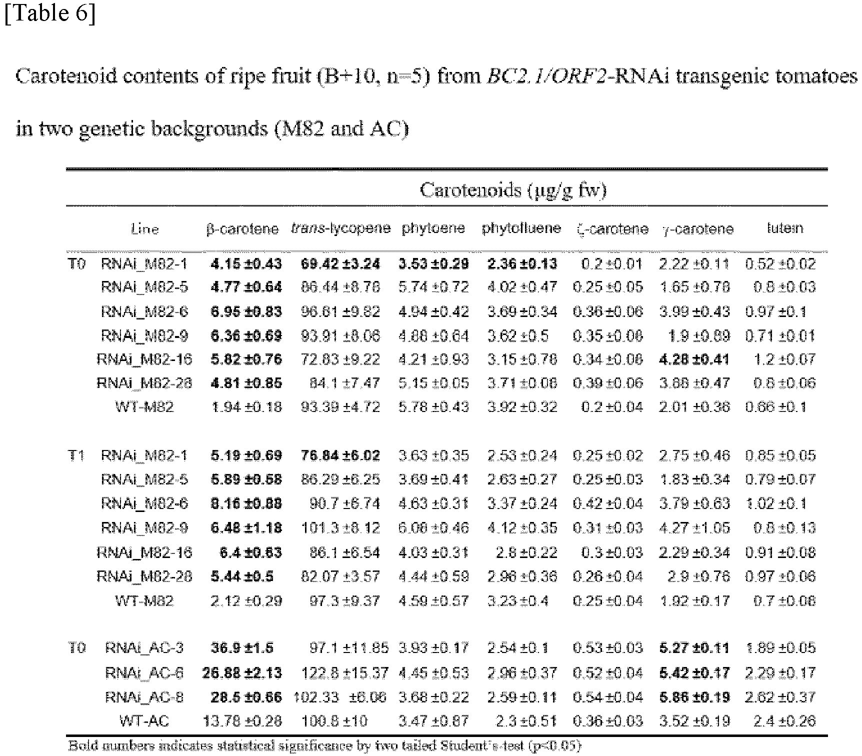

As described above, decreased expression of *ORF2* which was a SQE homolog affected the accumulation of fruit-specific carotenoids, which was suggested that there was a possible role in carotenoid metabolism. To study the molecular basis of *BC2.1*/*ORF2,* any genetic interaction with *Beta* was confirmed. SubIL2-2-1 was crossed with IL6-3 (including a *Beta* allele of *CYC-B*)*,* and one homozygous line (DIL26) in which both genes were introgressed was selected from the resulting F₂ population. beta-carotene constituted less than 5% of the total carotenoids in M82 and about 40% of the total carotenoids in red-orange *Beta* (IL6-3) fruits. On the other hand, DIL26 fruits were orange in color and beta-carotene accounted for almost 90% of total carotenoids, which had at least 24 times increased beta-carotene (FIG. 3A, Table 4). These results suggested that *bc2.1* had a genetically synergic effect with *Beta* for beta-carotene synthesis.

A carotenoid profile of the DIL26 genotype was consistent with a previously proposed two-gene model for beta-carotene content in tomato fruits, and in this model, beta-carotene was influenced by both *Beta* and a modifier gene referred to as a *Beta*-modifier (*MoB*). Moreover, DIL26 had a very similar profile of carotenoids to that reported in *S. galapagense* "LA317" (previously *Z. chesmannii*)*,* including *Beta* and *MoB* loci. To evaluate the intrinsic nature of the *bc2.1* allele in LA317, the promoter and cDNA sequences were analyzed. In the promoter, a 52 bp insertion having no obvious homology to previously known sequences was found in 1,234 bp upstream from the initiation codon (FIG. 7), and the insertion site was similar to that of the *Copia*-like retrotransposon found in the LA716 *bc2.1* allele. The *BC2.1*/*ORF2* expression in LA317 mature fruit was reduced to levels similar to those observed in subIL2-2-1 mature fruit, and the most abundant carotenoid in the orange-colored LA317 mature fruit was beta-carotene. In *S. cheesmaniae* "LA1412", which had an orange mature fruit color and showed a carotenoid profile similar to LA317 (Table 7), the same mutation was found (FIG. 10). Similar to DIL26, lycopene was not detected in the mature fruits of LA317 and LA1412 (FIG. 3C), which proposed *bc2.1* as a promising candidate for *MoB.*

**[Table 7]**

| **Carotenoid profiles of LA317 and LA1412 (n=5)** | | |
|---|---|---|
| Carotenoid | Carotenoid contents (µg/g fw) | |
| | LA317 | LA1412 |
| Phytoene | n.d. | 4.79 ± 0.29 |
| Phytofluene | n.d. | 2.87 ± 0.14 |
| Lutein | 1.86±0.14 | 1.19 ± 0.03 |
| β-carotene | 28.62±4.52 | 38.53 ± 0.87 |
| γ-carotene | n.d. | n.d. |
| *trans*-lycopene | n.d. | n.d. |

| | | |
|---|---|---|
| n.d.: not detected | | |

As derived a genetic interaction between *bc2.1* and *Beta,* subIL2-2-1 was crossed with IL12-2, which contains the *Delta* allele of lycopene e-cyclase (*LCY-E*)*,* to breed a double genetic introgression line referred to as DIL212. DIL212 represented orange-colored fruits when compared with IL12-2 (*Delta*)*,* which exhibited a deep orange color and M82, which exhibited a red color in mature fruits (FIG. 3B). A very small amount of lutein was accumulated in the mature fruit of M82, whereas lutein (11.74 ± 0.80 mg/g fw) in DIL212 was at least 20 times higher than that of M82. As seen in DIL26, a very low amount of lycopene was accumulated even in DIL212, which suggested a genetic interaction between *bc2.1* and *Delta.* Lutein mutations in IL were not observed, which was because *LCY-E* was not expressed in mature tomatoes including M82 (FIG. 1C).

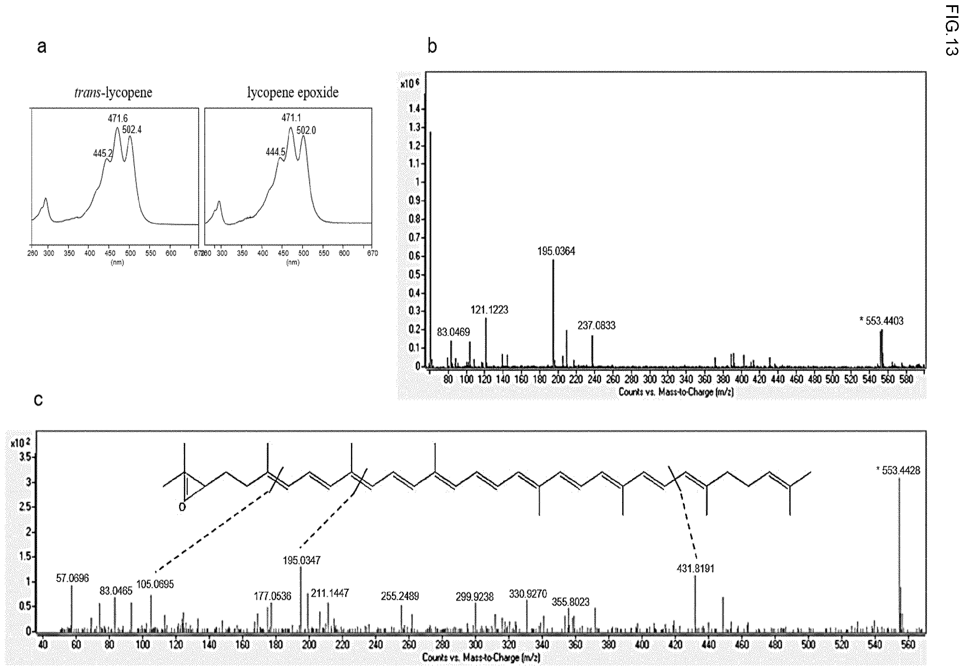

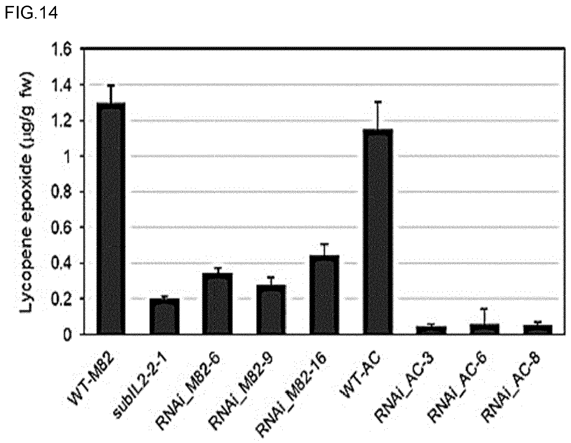

Based on an increase in beta-carotene content associated with BC2.1/ORF2, the possible activity of encoded enzymes in carotenoid metabolism was confirmed. The recombinant proteins (SlBC2.1 from M82 and SpBC2.1 from LA716) were introgressed into *E. coli,* which highly accumulated *trans*-lycopene previously constructed. In *trans*-lycopene accumulated *E*. *coli,* when SlBC2.1 (JX683513, 75 to 602 a.a.) or SpBC2.1 (JX683514, 75 to 601 a.a.) was expressed together with an expression vector pTrcHis2-TOPO, most of the *trans*-lycopene was converted to a new peak (FIG. 3E). Despite not being able to obtain a standard product, LC-MS/MS analysis found that a new peak with m/z of 553.4428 was protonated lycopene epoxide (FIGS. 12 and 13). Lycopene epoxide has been previously identified in tomato fruits and reported to be produced through catalytic and non-catalytic processes. Considering that lycopene epoxide has been identified in human plasma, its bioavailability remained a subject to be continuously studied. Lycopene epoxide was quantified by HPLC and decreased in RNAi line and subIL2-2-1 compared to M82 or AC (FIG. 14). These results indicated that BC2.1 was lycopene epoxidase leading to a new branch of a lycopene pathway (FIG. 3F).

Allelic mutations in a coding region of BC2.1/ORF2 (FIG. 2A) were not a cause of the *bc2.1* fruit phenotype. Instead, this phenotype was caused by a difference in expression of the *BC2.1*/*ORF2* allele. Although the *bc2.1* phenotype was limited to fruit, expression mutation was not limited to the fruit (FIG. 2C). Based on the lycopene epoxide activity of BC2.1/ORF2 shown in the *E. coli* system, the *bc2.1* phenotype was determined by reduced expression of the LA716 allele and by lycopene which was a mature fruit-specific *bc2.1* substrate.

As mentioned above, BC2.1 contained a predicted transport peptide (FIG. 7). A BC2.1-GFP fusion protein was transiently expressed in the leaves of *N. benthamiana* under the control of a CaMV 35S promoter and a sub-cellular location of BC2.1 was confirmed by confocal laser microscopy. A GFP fluorescence signal at 488 nm showed that the BC2.1-GFP fusion protein was localized to a chromatophore, probably a plastoglobule, whereas pCAMBIA2300-eGFP was localized to a nucleus (FIG. 8).

It was considered that the carotenoid pathway acted as a multi-enzyme complex to enable metabolite channeling, as would be expected in the absence of a pathway intermediate and in the presence of a complex comprising a carotenoid biosynthetic enzyme. Flag-labeled *BC2.1* (JX683513, 1 to 602 a.a.) and Myc-labeled *CYC-B* (AF254793, 1 to 498 a.a.) were transiently expressed in *N. benthamiana.* In immunoblot analysis using an αMyc antibody, BC2.1 migrated as a doublet; Thus, it was shown that BC2.1 had post-translational modifications (FIG. 3D). Co-immunoprecipitation analysis showed that BC2.1 formed a complex with *CYC-*B, but did not form a complex with GFP, which was used as a negative control (FIG. 3D), which suggested that BC2.1 was associated with *CYC-B in planta.*

In genetic and biochemical analysis of *bc2.1,* it was shown that the lycopene epoxide activity of *BC2.1*/*ORF2* competed with *CYC-B* for its substrate, *trans*-lycopene, and the balance of *BC2.1*/*ORF2* and *CYC-B* activities determined the relative amounts of lycopene and beta-carotene in mature fruits. Similarly, *BC2.1*/*ORF2* also competed with *LCY-E* in *Delta* to regulate a lutein level. A new target regulating provitamin A was identified by analyzing naturally occurring mutations in the *bc2.1* locus.

Nutritional problems are a major problem emerging internationally, and continuous research is required to solve the problems. Accordingly, there is an increasing demand for genetically improving the nutrients of many crop plants. In addition, it is possible to understand the genetic and biochemical basis of accumulation of plant carotenoids by identifying lycopene epoxidase and to provide a molecular tool for screening for allelic variants or genetic variants for increased provitamin A and lutein.

Hereinabove, the present invention has been described with reference to preferred exemplary embodiments thereof. It will be understood to those skilled in the art that the present invention may be implemented as a modified form without departing from an essential characteristic of the present invention. Therefore, the disclosed exemplary embodiments should be considered in an illustrative viewpoint rather than a restrictive viewpoint. The scope of the present invention is illustrated by the appended claims rather than by the foregoing description, and all differences within the scope of equivalents thereof should be construed as being included in the present invention.
Project Unique Number: 1711140395
Project Number: 2021R1A2C2093789
Ministry name: Ministry of Science and ICT
Project Management (Special) Institution Name: National Research Foundation of Korea
Research Business Name: Personal basic research (Ministry of Science and ICT) (R&D)
Research Subject Name: Study on novel tomato-pepper fruit ripening-dependent pigment control mechanism
Percent Contribution: 1/1
Project performance institute name: Kyungpook National University
Research Period: September 1, 2021 to February 28, 2022

## Claims

1. A tomato in which the expression of BC2.1 is inhibited and the amount of beta-carotene or lutein is increased.

2. The tomato in which the amount of beta-carotene or lutein is increased of claim 1, wherein the expression of ORF2 of the BC2.1 is inhibited.

3. The tomato in which the amount of beta-carotene or lutein is increased of claim 1, wherein the expression of the BC2.1 is inhibited by administering an RNAi construct.

4. The tomato in which the amount of beta-carotene or lutein is increased of claim 1, wherein the expression of the BC2.1 is inhibited by administering a sequence encoding BC2.1 containing a mutation to the tomato.

5. The tomato in which the amount of beta-carotene or lutein is increased of claim 4, wherein the sequence encoding BC2.1 containing the mutation is selected from nucleic acid sequences represented by SEQ ID NOs: 2 to 4.

6. The tomato in which the amount of beta-carotene or lutein is increased of claim 3, wherein the RNAi construct is prepared using RNAi-F having a sequence represented by SEQ ID NO: 59 and RNAi-R having a sequence represented by SEQ ID NO: 60.

7. The tomato in which the amount of beta-carotene or lutein is increased of claim 1, wherein the expression of the BC2.1 is inhibited to express *LCY-E.*

8. The tomato in which the amount of beta-carotene or lutein is increased of claim 1, wherein the tomato is obtained by crossing subIL2-2-1, a genetic introgression line of Solanum. pennellii LA716, with IL6-3, or produced by crossing subII,2-2-1 with IL12-2.
